# EUROPEAN PATENT APPLICATION

(11) **EP 4 545 627 A1**
(43) Date of publication of application: **30.04.2025**
(21) Application number: 23846762.5
(22) Date of filing: 15.05.2023
(51) Int. Cl.: C12N 1/18, A21D 8/04, C12R 1/865

(54) **NOVEL BREAD-MAKING YEAST SACCHAROMYCES CEREVISIAE SPC Y76LT WITH SUPERB FERMENTATION PROPERTY**

(30) Priority: 28.07.2022 KR 20220094198
(71) Applicant: SPC Co., Ltd., Seongnam-si, Gyeonggi-do 13220 (KR)
(72) Inventor: CHOI, Eun Ji, Seoul 08826 (KR); JUNG, Moon Young, Seoul 08826 (KR); OH, Hye Won, Seoul 08826 (KR); LEE, Jong Hyuk, Seoul 08826 (KR); SHIM, Sang Min, Seoul 08826 (KR); SEO, Jin Ho, Seoul 08826 (KR)
(74) Representative: RGTH
(86) International application number: PCT/KR2023/006551
(87) International publication number: WO 2024/025101

(57) **Abstract**

The present invention pertains to (Saccharomyces cerevisiae) SPC Y76LT (KCTC 14767BP). The yeast strain (SPC Y76LT) of the present invention exhibits excellent fermentation efficiency across various sugar contents and thus can be applied to a wide variety of breads with differing sugar levels. Furthermore, the strain of the present invention shows reduced fermentation activity at low temperatures, which leads to minimal quality changes during refrigerated distribution, and thus is suitable for refrigerated dough distribution. Additionally, the dough made using the strain of the present invention retains its superior fermentation capabilities even after being stored in a frozen state and then thawed, and thus is suitable for frozen dough distribution.

## Description

### [Technical Field]

The present invention relates to a novel yeast for baking, and more specifically, to a novel yeast *Saccharomyces cerevisiae* SPC Y76LT for baking that exhibits excellent fermentation characteristics at various sugar concentrations, excellent low-temperature sensitivity (minimized fermentation and metabolism at low temperatures), and superior freeze-storage properties.

### [Background Art]

There are various types of flour dough compositions for bread, such as French bread containing no sucrose like baguette, white bread containing a low amount of sucrose, and sweet bread containing a great amount of sucrose like sweet red bean bread.

There are two main bread production methods. One is a straight method of immediately reflecting the fermentation power of the bread yeast by kneading bread dough raw materials at once, followed by fermenting and baking.

The other is a sponge dough method based on a two-stage bread fermentation process. In other words, a sponge dough is first prepared and fermented, and then a final flour dough is prepared and fermented. The sponge dough method is widely used for baking because it has the advantages of increasing the bread volume by increasing the flexibility and gas retention capacity of the bread, improving the mechanical resistance of the flour dough, and improving the flavor of the bread.

Meanwhile, in modern times, various bread production and distribution methods are being introduced to establish an efficient mass production system (a manner in which bread produced in a factory is baked in a store) for supplying fresh bread, and typical examples thereof are the distribution of frozen dough and refrigerated dough. In consideration of the variety of types of bread and the variety of the characteristics of the production and distribution processes of bread, the selection of yeast that can best reflect each characteristic may be the most important factor in bread quality, and yeast with various characteristics are currently being developed and used commercially.

Bread yeast that has different fermentation characteristics depending on the sugar content is the most typical. Most bread yeast used in bread dough with low sucrose content has strong ability to utilize maltose in the flour used, and bread yeast with strong sugar (salt) tolerance is used for bread with high sucrose content and salt concentration. In addition, yeast with freezing tolerance or yeast that is sensitive to low temperatures is selected for use in frozen dough or refrigerated dough.

Yeast is involved in fermentation and is a key factor for baking that determines taste, aroma, and flavor. However, yeast developed and produced by some global companies has been imported or has been produced in Korea from purchased foreign yeast. Korean fermented foods have infinite value as a material for securing Korean fermentation microorganism resources. Much research has been conducted on the fermentation process, but research on microorganisms involved in fermentation is insufficient. There are few cases of utilizing Korean fermentation microorganisms as resources and thus national support itself is insufficient.

In order to achieve the success of fermentation in bread, improvement of yeast, which is a fermentation starter, is required in order for the yeast to adapt well to the baking environment. In addition, research on improvement of starter is required to increase the flavor and functionalities of bread for quality improvement and more market development.

### [Disclosure]

### [Technical Problem]

In the previous research, the native yeast (*Saccharomyces cerevisiae*), SPC-SNU 70-1, isolated from Korean traditional yeast, called "Nuruk", was used as an excellent bakery yeast due to excellent effect of improving quality such as taste, flavor, and anti-aging.

However, SPC-SNU 70-1 is disadvantageously unsuitable for use in European bread containing no sugar or sweet bread having a high sugar content due to the low fermentation power in bread with no or very low sugar and very high sugar. In addition, quality changes should be minimized during refrigerated and frozen distribution of flour dough. However, conventional SPC-SNU 70-1 strains had a problem in that fermentation proceeded even at low temperatures (10°C), thus making quality control difficult during distribution.

Therefore, the present invention provides a novel yeast that can produce dough not greatly depending on sugar content, exhibits excellent fermentation power at various sugar contents, and minimizes quality changes at refrigerated and frozen distribution temperatures.

### [Technical Solution]

In accordance with an aspect of the present invention, the above and other objects can be accomplished by the provision of *Saccharomyces cerevisiae* SPC Y76LT (KCTC 14767BP) having freeze-storage properties as well as low-temperature sensitivity.

In accordance with another aspect of the present invention, there is provided a dough for baking containing *Saccharomyces cerevisiae* SPC Y76LT (KCTC 14767BP) having freeze-storage properties as well as low-temperature sensitivity.

In accordance with a further aspect of the present invention, there is provided a bread produced by baking a dough for baking containing *Saccharomyces cerevisiae* SPC Y76LT (KCTC 14767BP) having freeze-storage properties as well as low-temperature sensitivity.

### [Advantageous effects]

The *Saccharomyces cerevisiae* SPC Y76LT (KCTC 14767BP) of the present invention exhibits excellent fermentation characteristics at various sugar concentrations, thus being widely utilized from bread with a low sugar content to bread with a high sugar content.

In addition, the *Saccharomyces cerevisiae* SPC Y76LT (KCTC 14767BP) of the present invention suppresses fermentation at refrigerated temperatures, thus being suitable for refrigerated dough distribution.

In addition, the dough prepared using the *Saccharomyces cerevisiae* SPC Y76LT (KCTC 14767BP) of the present invention has excellent refrigerated storage properties due to high trehalose content thereof. In other words, the dough maintains the excellent fermentation power of the strain although the dough is stored in a frozen state and then thawed, thus being suitable for distribution of frozen dough.

### [Description of Drawings]

FIG. 1 is a schematic diagram illustrating a process of developing *Saccharomyces cerevisiae* SPC Y76LT of the present invention.
FIG. 2 shows the difference in ITS sequence between *Saccharomyces cerevisiae* SPC Y76LT of the present invention and other yeasts.
FIGs. 3 and 4 show the difference in fermentation power depending on dough sugar concentration between *Saccharomyces cerevisiae* SPC Y76LT of the present invention and other yeasts.
FIG. 5 shows the measured fermentation power at low temperature (10°C) of *Saccharomyces cerevisiae* SPC Y76LT of the present invention and other yeasts, which indicates that SPC Y76LT has excellent low-temperature sensitivity and thus has low gas generation.
FIG. 6 shows the measured diameter of dough produced using *Saccharomyces cerevisiae* SPC Y76LT of the present invention and other yeasts after freezing storage and thawing at a refrigerator temperature for 8 hours, which indicates that the strain of the present invention (SPC Y76LT) has excellent low-temperature sensitivity and thus has little change in dough diameter.
FIG. 7 shows bread produced by producing dough using *Saccharomyces cerevisiae* SPC Y76LT of the present invention and other yeasts, and then repeatedly freezing and thawing the dough.
FIG. 8 shows the measured aging degree and specific volume of the bread of FIG. 7.
FIG. 9 shows the measured fermentation power of dough that is produced using *Saccharomyces cerevisiae* SPC Y76LT of the present invention and other yeasts, freeze-stored for each period of time, and then fermented, which indicates that the dough using the strain of the present invention (SPC Y76LT) has excellent freeze-storage properties and high fermentation power.
FIG. 10 shows the measured time required for secondary fermentation of the dough that is produced using *Saccharomyces cerevisiae* SPC Y76LT of the present invention and other yeasts, and freeze-stored for each period of time, and the measured height of bread produced using the frozen dough, and indicates that the dough using the strain of the present invention (SPC Y76LT) has excellent freezing stability, has a short secondary fermentation time, and is produced into bread with a great height.

### [Best Mode]

The present invention provides *Saccharomyces cerevisiae* SPC Y76LT (KCTC 14767BP). In addition, the present invention provides dough and bread produced using the *Saccharomyces cerevisiae* SPC Y76LT (KCTC 14767BP).

In the previous research (Korean Patent No. 10-1551839), the native yeast *(Saccharomyces cerevisiae),* SPC-SNU 70-1, isolated from Korean traditional yeast, called "Nuruk", was used as an excellent bakery yeast due to excellent effect of improving quality such as taste, flavor, and anti-aging.

However, SPC-SNU 70-1 is disadvantageously unsuitable for use in various types of bread due to the low fermentation power in bread with no or very low sugar and very high sugar. However, dough using the SPC-SNU 70-1 strain has poor low-temperature sensitivity, thus being disadvantageously unsuitable for distribution in the form of refrigerated dough.

On the other hand, the Y76LT of the present invention exhibits excellent fermentation power regardless of the sugar content of the dough and thus is useful for production of a wider variety of breads.

In addition, the SPC Y76LT of the present invention has excellent low-temperature sensitivity. Therefore, fermentation of the dough using the strain of the present invention is inhibited during the refrigerated distribution process and changes in the quality of the dough are minimized.

In addition, the SPC Y76LT of the present invention has excellent trehalose productivity. Therefore, the dough using the strain of the present invention has excellent refrigeration storage properties. That is, although the dough using the strain of the present invention that has been freeze-stored and then thawed is used for bread production, the excellent fermentation power of the strain is maintained.

Trehalose is known to play an important role in stress response to withstand moisture deficiency or freezing situations because it is synthesized as an energy source and has the property of retaining water. Therefore, the trehalose productivity of a strain is a parameter to determine the shelf life of the yeast, the freezing tolerance of the dough produced using the yeast, and the high sugar tolerance.

Hereinafter, the present invention will be described in more detail with reference to the following examples and experimental examples, but the scope of the present invention is not limited to the examples and experimental examples and includes variations and technical concepts equivalent thereto.

### [Example 1: Selection of Saccharomyces cerevisiae SPC Y76LT]

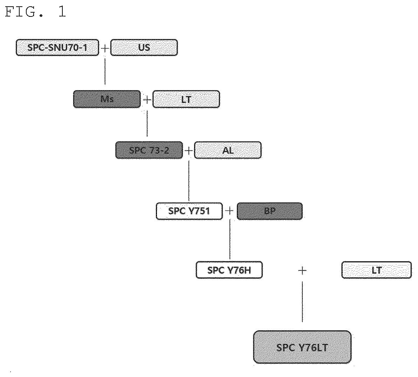

*Saccharomyces cerevisiae* SPC-SNU 70-1 (KCTC 12776BP, Korean Patent No. 10-1551839) discovered from Korean traditional yeast, called "Nuruk" was isolated from tetrads of various yeasts and then mated to develop a novel yeast (FIG. 1). The developed strain was named "SPC Y76LT" and deposited with the Korea Research Institute of Bioscience and Biotechnology (on November 10, 2021) and assigned the deposit number "KCTC 14767BP".

FIG. 1 is a schematic diagram showing a process of developing *Saccharomyces cerevisiae* SPC Y76LT of the present invention.

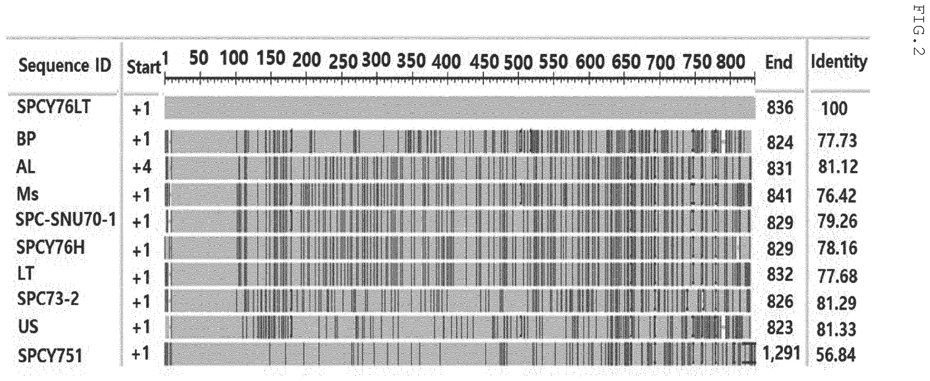

Meanwhile, the sequence differences in the ITS (internal transcribed spacer) region between the yeasts used in the development process (FIG. 2) were identified. The result showed that *Saccharomyces cerevisiae* SPC Y76LT had genetic differences from various yeasts used in the mating process. This means that *Saccharomyces cerevisiae* SPC Y76LT of the present invention is a novel yeast different from the yeasts used in the mating.

Meanwhile, in FIG. 2, the parts of *Saccharomyces cerevisiae* SPC Y76LT sequence that differ from the sequence of yeast used for mating are marked in red.

### [Experimental Example 2: Experiment to identify differences in biochemical characteristics between respective yeasts through VITEK-2 analysis]

In this example, the differences in biochemical characteristics of the present invention *Saccharomyces cerevisiae* SPC Y76LT and various yeasts were identified through VITEK-2 analysis (Table 1 and Table 2).

**[Table 1]**

| Menemonic | Test | Yeast | | | | | | | | | |
|---|---|---|---|---|---|---|---|---|---|---|---|
| | | SPC-SNU7 0-1 | SPC73 -2 | SPCY 751 | US | LT | AL | BP | Ms | SPC7 6H | SPC76 LT |
| LysA | L-Lysine-ARYLAMIDAS E | | | | | | | | | | |
| lMLTa | L-MALTATE assimilati on | | | | | | | | | | |
| LeuA | Leucine-ARYLAMIDAS E | + | + | + | + | + | + | + | + | + | + |
| ARG | ARGININE GP | | | | | | | | | | |
| ERYa | ERYTHRITOL assimilati on | | | | | | | | | | |
| GLYLa | GLYCEROL assimilati on | | | | | | | | | | |
| TyrA | Tyrosine ARYLAMIDAS E | + | | | | | | | | | |
| BNAG | BETA-N-ACETYL-GLUCOSAMIN IDASE | | | | | | | | | | |
| ARBa | ARBUTIN assimilati on | | | | | | | | | | |
| AMYa | AMYGDALIN assimilati on | | | | | | | | | | |
| dGALa | D-GALACTOSE assimilati on | + | + | + | + | + | + | + | + | + | + |
| GENa | GENTIOBIOS E assimilati on | | | | | | | | | | |
| dGLUa | D-GLUCOSE assimilati on | + | + | + | + | + | + | + | + | + | + |
| LACa | LACTOSE assimilati on | | | | | | | | | | |
| MAdGa | METHYL-A-D-GLUCOPYRAN OSIDE assimilati on | | | + | | | + | | | | + |
| dCELa | D-CELLOBIOSE assimilati on | | | | | | | | | | |
| GGT | GAMMA-GLUTAMYL-TRANSFERAS E | | | | | | | | | | |
| dMAL a | D-MALTOSE assimilati on | + | + | + | + | + | + | + | + | + | + |
| dRAFa | D-RAFFINOSE assimilati on | + | + | + | + | + | + | + | + | + | + |
| NAGA1 | PNP-N-acetyl-BD-galactosar ninidase 1 | | | | | | | | | | |
| dMNE a | D-MANNOSE assimilati on | + | + | + | + | + | + | + | + | + | + |
| dMELa | D-MELIBIOSE assimilati on | | | | | | | | | | |
| dMLZa | D-MELEZITOSE assimilati on | | | + | | | + | | | | |
| lSBEa | L-SORBOSE assimilati on | | | | | | | | | | |
| lRHAa | L-RHAMNOSE assimilati on | | | | | | | | | | |
| XLTa | XYLITOL assimilati on | | | | + | + | | + | | | + |

**[Table 2]**

| Menemonic | Substrat e | Yeast | | | | | | | | | |
|---|---|---|---|---|---|---|---|---|---|---|---|
| | | SPC-SNU70 -1 | SPC73 -2 | SPCY7 51 | US | LT | AL | BP | Ms | SPC7 6H | SPC7 6LT |
| dSORa | D-SORBITOL assimila tion | | | | | | | | | | |
| SACa | SACCHARO SE/SUCRO SE assimila tion | + | + | + | + | + | + | + | + | + | + |
| URE | UREASE | | | | | | | | | | |
| AGLU | ALPHA-GLUCOSID ASE | + | + | + | + | + | + | + | | + | + |
| dTURa | D-TURANOSE assimila tion | + | + | + | + | + | + | + | + | + | + |
| dTREa | D-TREHALOS E assimila tion | | + | + | + | + | + | + | + | + | + |
| NO3a | NITRATE assimila tion | | | | | | | | | | |
| IARAa | L-ARABINOS E assimila tion | | | | | | | | | | |
| dGATa | D-GALACTUR ONATE assimila tion | | | | | | | | | | |
| ESC | ESCUUN hydrolys is | | | | | | | | | | |
| lGLTa | L-GLUTAMAT E assimila tion | | | | | | | | | | |
| dXYLa | D-XYLOSE assimila tion | | | | | | | | | | |
| LATa | DL-LACTATE assimila tion | + | | | | | | | + | | |
| ACEa | ACETATE assimila tion | + | + | + | + | + | + | + | + | + | + |
| CITa | CITRATE ( SODIUM) assimila tion | | | | | | | | | | |
| GRTas | CLUCURON ATE assimila tion | | | | | | | | | | |
| IPROa | L-PROLINE assimila tion | | | | | | | | | | |
| 2KGa | 2-KETO-D-GLUCONAT E assimila tion | | | | | | | | | | |
| NAGa | N-ACETYL-GLUCOSAM INE assimila tion | | | | | | | | | | |
| dGNTa | D-GLUCONAT E assimila tion | | | | | | | | | | |

As can be seen from Tables 1 and 2, the *Saccharomyces cerevisiae* SPC Y76LT of the present invention exhibits different biochemical characteristics from the strains used for mating in Example 1, which means that the *Saccharomyces cerevisiae* SPC Y76LT of the present invention is a novel yeast different from the strains used for mating.

### [Example 3: Obtaining Saccharomyces cerevisiae SPC Y76LT]

1) The *Saccharomyces cerevisiae* SPC Y76LT of the present invention was inoculated into sterilized YM (yeast malt) medium and then was cultured at 30°C for 24 hours.
2) The culture product obtained in 1) was inoculated in 100 ml of sterilized YPD (yeast peptone dextrose) medium and then was cultured at 30°C for 24 hours.
3) 50 ml of the culture product obtained in 2) in 500 ml of sterilized YPD medium and then was cultured at 30°C for 24 hours.
4) The culture product obtained in 3) was inoculated into a culture medium containing molasses, urea, phosphate, and vitamin B using a jar-fermenter and then was cultured at 30°C for 24 hours.
5) 60 g of the cultured strain cells obtained by centrifuging the culture product obtained in 4) was inoculated in culture medium containing molasses, urea, phosphate, and vitamin B using a jar-fermenter and then was cultured at 30°C for 14 to 16 hours.
6) The culture product obtained in 5) was centrifuged, washed, and dehydrated to obtain yeast having a solid content of 34% (w/w).

### [Experimental Example 1: Confirmation of difference in sugar fermentation ability of Saccharomyces cerevisiae SPC Y76LT depending on sugar concentration change and temperature change]

In this experimental example, the sugar fermentation ability difference depending on the sugar concentration change and temperature change of the strain of the present invention, *Saccharomyces cerevisiae* SPC Y76LT, was determined.

### 1) Determination of difference in sugar fermentation power depending on the change in sugar concentration

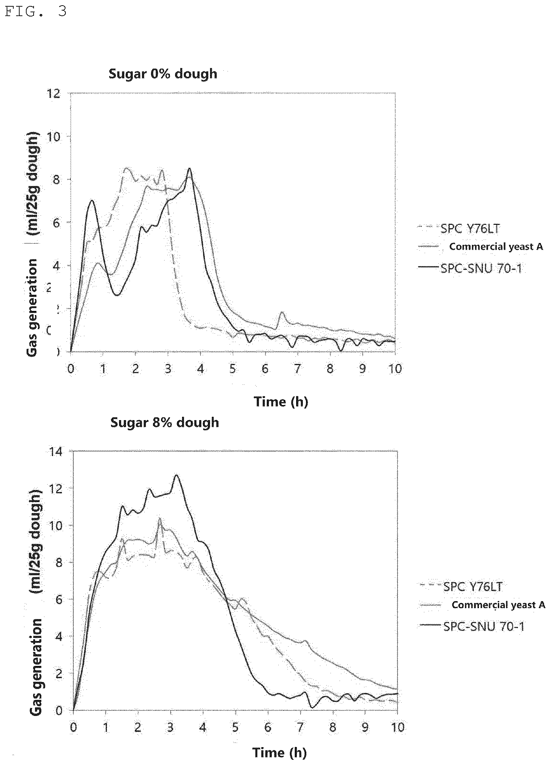

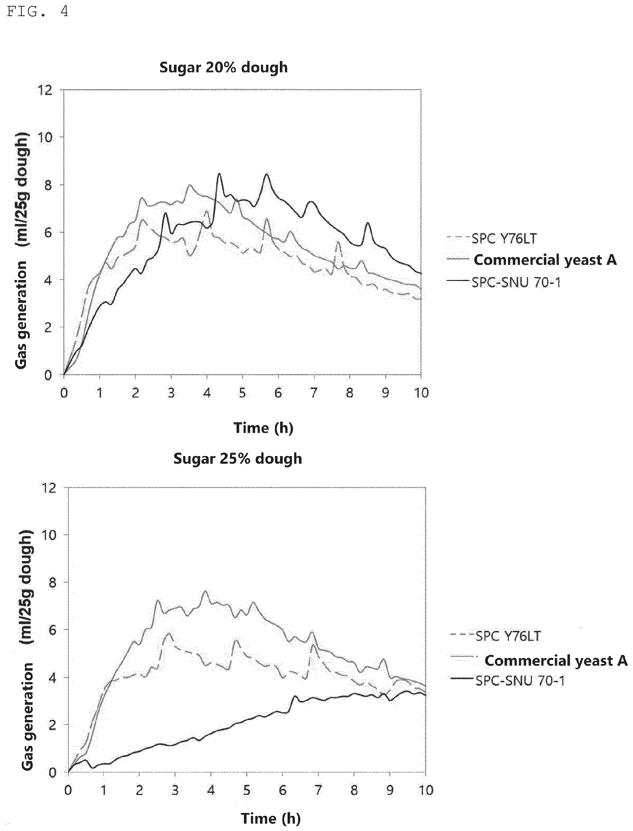

Dough with the composition ratio of Table 3 below was produced and then the fermentation power of 25 g of dough was measured using a gas generation power meter at a temperature of 30°C (FIG. 3 and FIG. 4).

**[Table 3]**

| Ingredient | Sugar 0% dough (g) | Sugar 8% dough (g) | Sugar 20% dough (g) | Sugar 25% dough (g) |
|---|---|---|---|---|
| Strong flour | 100 | 100 | 100 | 100 |
| Sugar | 0 | 8 | 20 | 25 |
| Skimmed milk powder | 0 | 4 | 4 | 4 |
| Refined salt (salt) | 2 | 2 | 2 | 2 |
| Shortening | 3 | 3 | 3 | 3 |
| Water | 65 | 65 | 53.75 | 53.75 |
| Yeast | 3.15 | 3.37 | 3.40 | 3.40 |

As can be seen from FIGs. 3 and 4, the *Saccharomyces cerevisiae* SPC Y76LT of the present invention has excellent fermentation power regardless of sugar concentration.

### (2) Determination of difference in sugar fermentation power depending on temperature change

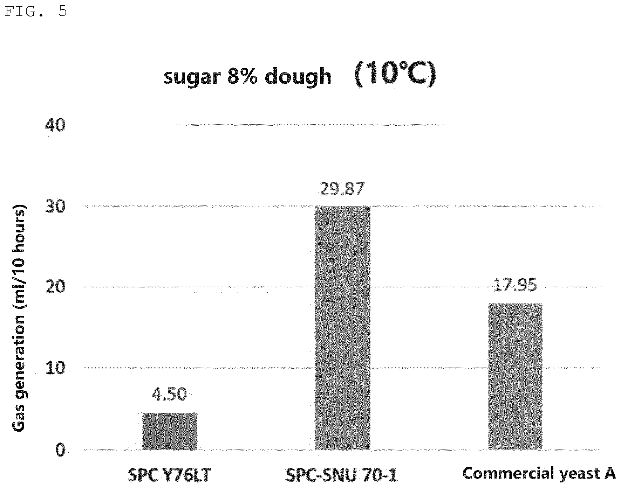

8% sugar dough (Table 3) was produced, the amount of gas generated from 25 g of the dough was measured for 10 hours using a gas generation power meter at a temperature of 10°C (FIG. 5).

Comparing FIG 3 with FIG. 5, it can be seen that the strain of the present invention (SPC Y76LT) has high low temperature sensitivity and the fermentation power is lowered at a low temperature (10 ° C) . This means that, although dough produced using the strain of the present invention (SPC Y76LT) is distributed in a refrigerated state, quality changes will be minimized.

### [Experimental Example 2: Experiment to determine low-temperature sensitivity of Saccharomyces cerevisiae SPC Y76LT]

In this experimental example, the low temperature sensitivity of *Saccharomyces cerevisiae* SPC Y76LT of the present invention was determined.

To this end, frozen dough was prepared using the strain of the present invention (SPC Y76LT), the diameter of the dough was measured, and the diameter of the dough after thawing was measured to determine whether there was any change in the diameter. In addition, bread was produced using the repeatedly frozen and thawed dough and the baking characteristics of the bread were determined. The specific experimental method and results thereof are as follows.

### 1) Dough preparation

The dough composition ingredients in Table 4 were added to a mixer (SK101S MIXER) and stirred for 5 minutes at low speed and 5 minutes at high speed to prepare dough. The prepared dough was aged in a fermenter at 27°C and 85% relative humidity for 10 minutes and was divided into equal sizes. Then, the dough was frozen in a quick freezer (-30°C) for 1 hour, and then stored in a frozen state at -18°C.

Meanwhile, in order to add yeast, the yeast (SPC Y76LT) having a solid content of 34% (w/w) prepared in Example 3 was used and yeast products of SPC 70-1 and commercial yeast A were used as control groups. At this time, the dough was prepared while changing the amount of water and yeast added in consideration of the solid content of each yeast product.

**[Table 4]**

| Ingredient | Weight rate |
|---|---|
| Strong flour | 100 |
| Modifier | 1.0 |
| Monoglyceride | 0.3 |
| Refined salt | 2.0 |
| Refined sugar | 14 |
| Skimmed milk powder | 3 |
| Butter | 10 |
| Milk | 20 |
| Yeast | 2.0 |
| Water | 44 |

### 2) Experiment to measure diameter change

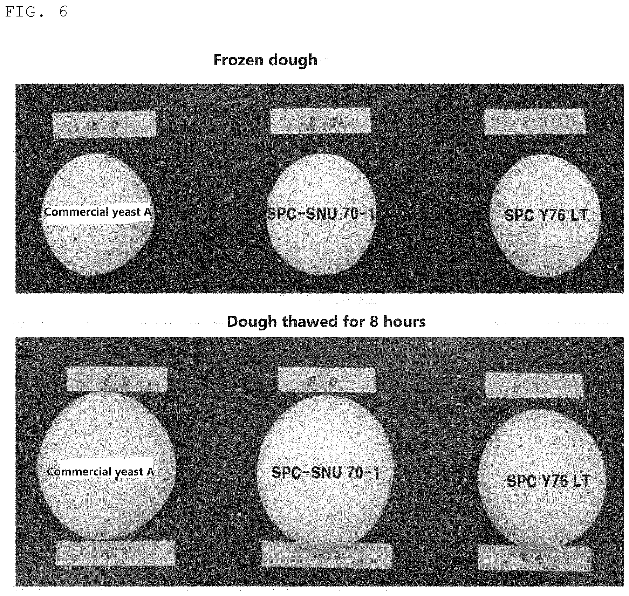

The diameter of the frozen dough prepared in 1) Dough preparation was measured and the diameter of the dough was measured after thawing at 10°C for 8 hours (FIG. 6).

As can be seen from FIG. 6, the dough prepared using the strain of the present invention (SPC Y76LT) had the smallest diameter change, from 8.1 cm to 9.4 cm. This means that the strain of the present invention (SPC Y76LT) has excellent low-temperature sensitivity and thus has low fermentation power under low temperature conditions, and that the dough prepared using the strain of the present invention (SPC Y76LT) has minimal quality changes although distributed in a refrigerated state.

### 3) Experiment to determine baking characteristics

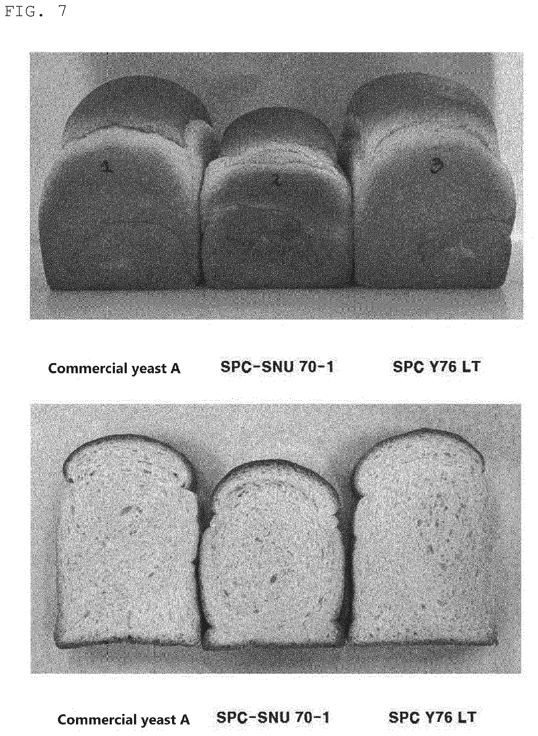

The frozen dough prepared in 1) Dough preparation was repeatedly frozen and thawed three times (thawed at room temperature for 1 hour and frozen at -18°C for 1 hour). The dough was thawed at 4 ° C for 12 hours, was exposed to room temperature for 1 hour to allow the dough temperature to be 18 ° C, rounded and placed in a bread case. The dough was fermented in a fermenter for 70 minutes at 35°C and 85% relative humidity. The dough was placed in an oven and baked for 35 minutes at 170°C on the upper heat and 210°C on the lower heat to produce bread (FIG. 7).

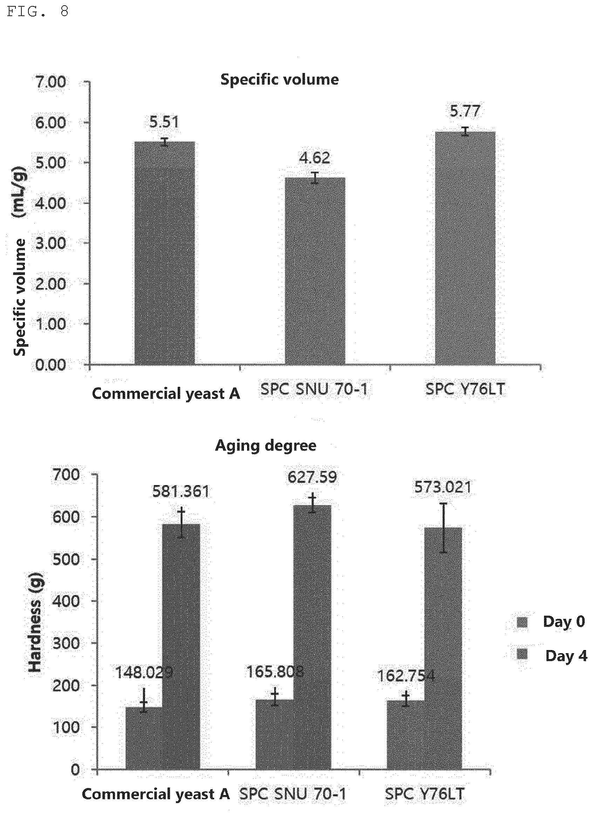

The produced bread was cooled at room temperature for about 2 hours, the pH, TTA (acidity), and moisture content of the bread were measured, and a sensory test was performed to obtain texture and flavor scores (Table 5). In addition, the aging degree and specific volume were measured using a specific volume meter and a physical property meter (FIG. 8).

**[Table 5]**

| Item | Commercial yeast A | SPC-SUN 70-1 | SPC Y76LT |
|---|---|---|---|
| pH | 5.41 | 5.49 | 5.40 |
| TTA(6.6/8.5) | 2.33/4.96 | 2.20/4.79 | 2.36/4.99 |
| Moisture content (%) | 39.33% | 39.23% | 39.27% |
| Texture | 8.0 | 6.5 | 8.5 |
| Flavor | 7.6 | 8.0 | 8.2 |

As can be seen from FIG. 7, the bread produced using the strain of the present invention (SPC Y76LT) has the greatest height.

Also, as can be seen from FIG. 8, the bread produced using the strain of the present invention (SPC Y76LT) has the greatest specific volume. Meanwhile, with respect to aging, the aging rate was measured immediately after the bread was produced and after 4 days. The result showed that the bread produced using the strain of the present invention (SPC Y76LT) has the lowest aging rate.

Meanwhile, according to the sensory evaluation, the two yeasts (SPC-SUN 70-1, SPC Y76LT) other than commercial yeast had a softer texture and better flavor. SPC Y76LT was found to have the best texture, which is considered to be due to the difference in specific volume.

### [Experimental Example 3: Experiment to confirm freezing stability of Saccharomyces cerevisiae SPC Y76LT]

In this experimental example, the excellent freezing stability of *Saccharomyces cerevisiae* SPC Y76LT of the present invention was confirmed.

To this end, the trehalose productivity of the strain of the present invention (SPC Y76LT) was measured. In addition, the dough prepared in Experimental Example 2 1) was freeze-stored and the difference in fermentation power and the difference in cost of the produced bread were measured depending on the frozen storage time.

### 1) Experiment to measure trehalose productivity

The yeast (SPC Y76LT) having a solid content of 34% (w/w) obtained in Example 3 was diluted with distilled water, and heated at 85 ° C for 25 minutes to extract trehalose. The extract was cooled to room temperature and then centrifuged, the result was filtered through a 0.45 µm nylon membrane filter, and the trehalose content was measured using a liquid chromatography-differential refractive detector. The concentrated strain (SPC Y76LT) culture solution of the present invention had a trehalose content of 13.58% (w/w) and thus excellent productivity.

Meanwhile, trehalose is known to play an important role in stress response to withstand moisture deficiency or freezing situations because it is synthesized as an energy source and is capable of retaining water.

### 2) Experiment to measure difference in fermentation power depending on dough freezing storage time

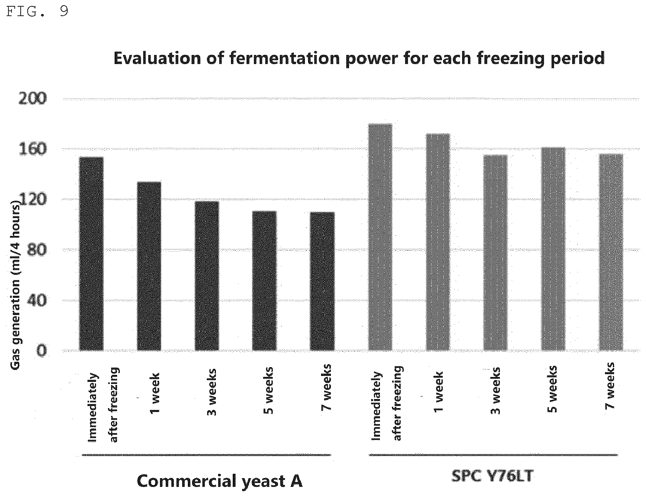

The dough prepared in 1) of Experimental Example 2 was frozen (1 week, 3 weeks, 5 weeks, 7 weeks). Then, the amount of gas generated from 25 g of dough was measured for 4 hours using a gas generation power meter at a temperature of 30°C (FIG. 9).

As can be seen from FIG. 9, the strain (SPC Y76LT) of the present invention has excellent freezing storage properties and thus maintains excellent fermentation power although the dough is stored in a frozen state for a long period of time, resulting in a high gas generation amount.

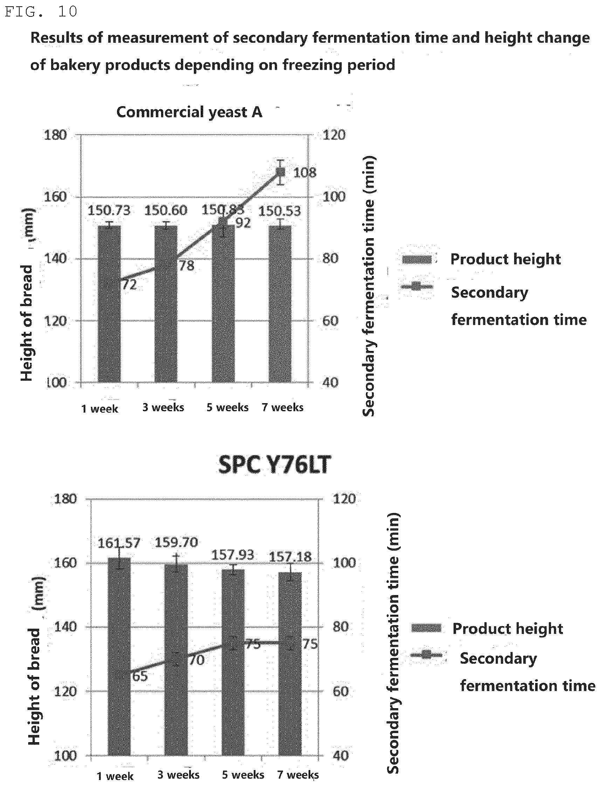

In addition, the dough prepared in 1) of Experimental Example 2 was frozen (1 week, 3 weeks, 5 weeks, 7 weeks), and then fermented in a fermenter at 35°C and 85% relative humidity, and the time until the height of the dough reached 95% of the height of the bread case was measured (FIG. 10). As can be seen from FIG. 10, the dough maintained excellent fermentation power although it was stored in a frozen state for a long period of time, and thus less time was required for fermentation.

### 3) Experiment to measure difference in height of bread products depending on dough frozen storage time

The dough prepared in 1) of Experimental Example 2 was frozen (1 week, 3 weeks, 5 weeks, 7 weeks). Then, bread was produced with the dough. The produced bread was cooled at room temperature for about 2 hours and the height of the bread was measured (FIG. 10).

As can be seen from FIG. 10, the finished product of bread using the strain of the present invention (SPC Y76LT) has a great height. This is because the bread using the strain of the present invention (SPC Y76LT) has excellent fermentation power although the dough is stored in a freezer for a long time.

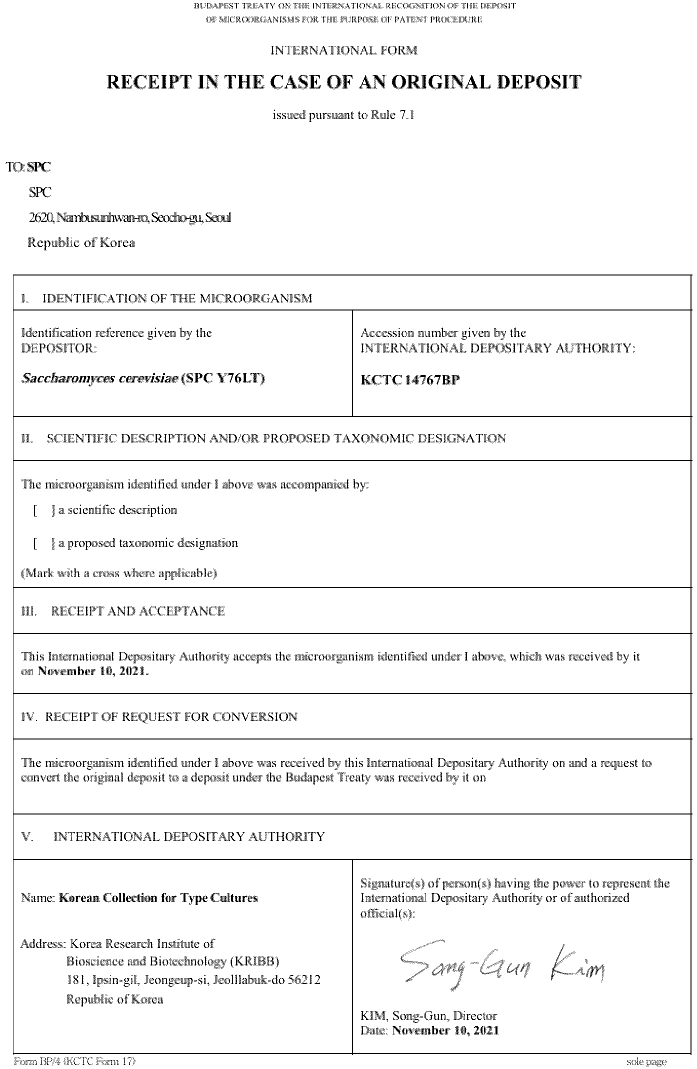
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: SPC
SPC
2620, Nambusunhwan-ro, Seocho-gu, Seoul
Republic of Korea

I. IDENTIFICATION OF THE MICROORGANISM
Identification reference given by the
DEPOSITOR:

Saccharomyces cerevisiae (SPC Y76LT) | Accession number given by the
INTERNATIONAL DEPOSITARY AUTHORITY:

KCTC 14767BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on November 10, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: November 10, 2021
Form BP/4 (KCTC Form 17) sole page

## Claims

1. *Saccharomyces cerevisiae* SPC Y76LT (KCTC 14767BP) having freeze-storage properties as well as low-temperature sensitivity.

2. A dough for baking containing *Saccharomyces cerevisiae* SPC Y76LT (KCTC 14767BP) having freeze-storage properties as well as low-temperature sensitivity.

3. A bread produced by baking a dough for baking containing *Saccharomyces cerevisiae* SPC Y76LT (KCTC 14767BP) having freeze-storage properties as well as low-temperature sensitivity.
